# EUROPEAN PATENT APPLICATION

(11) **EP 3 578 187 A1**
(43) Date of publication of application: **11.12.2019**
(21) Application number: 18748532.1
(22) Date of filing: 30.01.2018
(51) Int. Cl.: A61K 35/15, A61K 8/98, A23L 33/10, A61Q 19/00

(54) **PHARMACEUTICAL COMPOSITION FOR PREVENTING OR TREATING HYPERSENSITIVITY IMMUNE DISEASE, AND METHOD FOR PRODUCING SAME**

(30) Priority: 31.01.2017 KR 20170014075
(71) Applicant: Quratis Inc., Seoul 08375 (KR)
(72) Inventor: SHIN, Sung Jae, Seoul 06291 (KR); CHOI, Wahn Soo, Seoul 06291 (KR); KIM, Woo Sik, Seoul 03722 (KR); KIM, Hyuk Soon, Seoul 05333 (KR); KIM, Hong Min, Seoul 03722 (KR)
(74) Representative: Cabinet Becker et Associés
(86) International application number: PCT/KR2018/001295
(87) International publication number: WO 2018/143650

(57) **Abstract**

The present invention relates to a production method of a composition that can prevent, ameliorate, or treat hypersensitivity immune diseases including allergic diseases, and the composition produced by the method. In the method, the plasmacytoid dendritic cells with tolerogenic capacity may be induced from the immature dendritic cells at a high yield using a simple and easy process. The plasmacytoid dendritic cells can effectively prevent, ameliorate or treat the hypersensitivity immune diseases

## Description

### CROSS-REFERENCE TO RELATED APPLICATIONS

This application is based on and claims priority from Korean Patent Application No. 10-2017-0014075, filed on January 31, 2017, with the Korean Intellectual Property Office, the disclosure of which is incorporated herein in its entirety by reference.

### TECHNICAL FIELD

The present disclosure relates to a production method of a composition that can prevent, ameliorate, or treat hypersensitivity immune diseases including allergic diseases, and to the composition produced by the method.

### BACKGROUND

In the immune system, the immune response and the immune tolerance reaction must be coordinated and balanced with each other. When an over-immune response occurs, for example, hypersensitivity allergic diseases or autoimmune disease may occur. They have a great impact on human survival and quality of life.

Allergy refers to a pathological hypersensitivity reaction caused by an immunological mechanism against non-self-substances such as allergens, and is mediated by antibodies or cells. The allergic reaction is represented by T-helper type 2 (Th2) cell reaction which involves cytokines such as IL-4, IL-5, IL-13 and tumor necrosis factor (TNF)-a. chemokines such as Regulated upon activation, normal T-cell expressed & secreted (RANTES) eotaxin and macrophage chemoattractant protein-3 (MCP-3) play an important role in the allergic inflammatory response. Further, autoimmune diseases (autoimmunity) that show pathological hypersensitivity to self-substances are included therein.

Diseases caused by allergic reactions include asthma, rhinitis, urticaria, anaphylaxis, and the like. Some allergic reactions include allergic asthma and eczema caused by genetic linkage to specific allergens. These allergies are characterized by being caused generally by harmless substances (pollen, fungus, animal hair and dandruff, dust, mites, peanuts, and the like).

Currently developed treatments method for the allergic disease may include a method of identifying an allergen causing allergy which an allergic patient is currently suffering from, administering the allergen in small amounts for several years, and gradually reducing the allergy. However, this treatment method has disadvantages that the treatment period takes several years, and anaphylactic shock, and the like may be caused. In addition to this method, there are a method using DNA vaccine, a treatment method in which IgE is prevented from binding to the receptor of obesity cell, and an antibody treatment against IL-4 which is an allergen-inducing cytokine. However, these treatments are either costly or the effects thereof have not yet been fully demonstrated.

Meanwhile, dendritic cells play a pivotal role not only in innate immunity but also in controlling acquired immunity, which is selectively induced by acquired causation. Specifically, the dendritic cells are a type of an antigen presenting cell that performs a function of presenting information about an antigen mostly invaded from the outside to T cells.

The dendritic cells are composed of various subpopulations depending on their origin, phenotype, and function. In particular, plasmacytoid dendritic cells (pDC) may produce I-type IFN at high levels in response to stimuli derived from various pathogens. The plasmacytoid dendritic cells are distinguished from conventional dendritic cells (cDCs) based on the surface phenotypes. The pDC expresses B220, and expresses CD11c at a low level. The cDC expresses both CD11c and CD11b at high levels, and does not express B220. Both the pDC and cDC are obtained from a process by which the immature dendritic cells are mature so that the pDC and cDC can act as effective stimulants for a harmonious immune response.

As noted, pDC is known to play an important role in defense against virus infection by activating immune cells via secretion of large amounts of type I IFNs upon infection by DNA viruses or single-stranded RNA viruses. In particular, pDC strongly activates mature dendritic cells (mDCs) capable of presenting antigens, thereby helping to amplify antiviral responses by T cells.

However, studies on the differentiation or role of pDC in the body have been not vigorous compared to other dendritic cells. There is an urgent need for the production technology of tolerogenic dendritic cells that maintain or enhance immunity tolerance consistently by controlling the expression of specific genes responsible for the immunity of the dendritic cells or discovering immune tolerance inducing substances. In particular, although there are many studies that pDCs play an important role in various hypersensitivity immune diseases, a systematic and standardized differentiation method for producing tolerogenic pDCs is unknown.

### SUMMARY

The inventors of the present disclosure discovered that treatment of a toll-like receptor agonist (TLR agonist) to the immature dendritic cells to induce the differentiation of the immature dendritic cells or treatment of the toll-like receptor agonist during differentiating the immature dendritic cells results in inducing tolerogenic plasmacytoid dendritic cells, and then, the hypersensitivity immune diseases could be effectively prevented, ameliorated or treated using the tolerogenic plasmacytoid dendritic cells. Thus, the present disclosure has been achieved.

A purpose of the present disclosure is to provide a method for producing a composition that can effectively prevent, ameliorate, or treat hypersensitivity immune diseases.

Another purpose of the present disclosure is to provide a composition which may be produced by the aforementioned method and that can effectively prevent, ameliorate, or treat hypersensitivity immune diseases.

Other purposes and advantages of the present disclosure will become more apparent from the following detailed description of the invention, claims and drawings.

An exemplary embodiment of the present disclosure provides a production method of a pharmaceutical composition for prevention or treatment of hypersensitivity immune diseases, the method including producing tolerogenic plasmacytoid dendritic cells (pDC) by treating the immature dendritic cells with toll-like receptor agonists.

Further, another exemplary embodiment of the present disclosure provides a pharmaceutical composition for prevention or treatment of hypersensitivity immune diseases, the composition including tolerogenic plasmacytoid dendritic cells induced by treating the immature dendritic cells with toll-like receptor agonists.

Furthermore, still another exemplary embodiment of the present disclosure provides a use for the prevention or treatment of hypersensitivity immune diseases, of tolerogenic plasmacytoid dendritic cells induced by applying toll-like receptor agonists to immature dendritic cells.

Furthermore, still another exemplary embodiment of the present disclosure provides a use for production of a pharmaceutical composition for prevention or treatment of hypersensitivity immune diseases, of tolerogenic plasmacytoid dendritic cells induced by applying toll-like receptor agonists to immature dendritic cells.

Furthermore, still another exemplary embodiment of the present disclosure provides a cosmetic composition for the prevention or amelioration of hypersensitivity immune diseases, the composition including tolerogenic plasmacytoid dendritic cells induced by applying toll-like receptor agonists to immature dendritic cells.

Furthermore, still another exemplary embodiment of the present disclosure provides a food composition for the prevention or amelioration of hypersensitivity immune diseases, the composition including tolerogenic plasmacytoid dendritic cells induced by applying toll-like receptor agonists to immature dendritic cells.

According to the exemplary embodiments of the present disclosure, the tolerogenic plasmacytoid dendritic cells may be induced from the immature dendritic cells at a high yield using a simple and easy process, thus enabling stable supply of a large number of the tolerogenic plasmacytoid dendritic cells.

Further, according to the exemplary embodiments of the present disclosure, the tolerogenic plasmacytoid dendritic cells obtained as described above can effectively prevent or treat various hypersensitivity immune diseases including food-derived allergy disease by inhibiting expression of allergen-specific immunoglobulins and Th2 cytokines. The tolerogenic plasmacytoid dendritic cells are safe when administered to human body.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

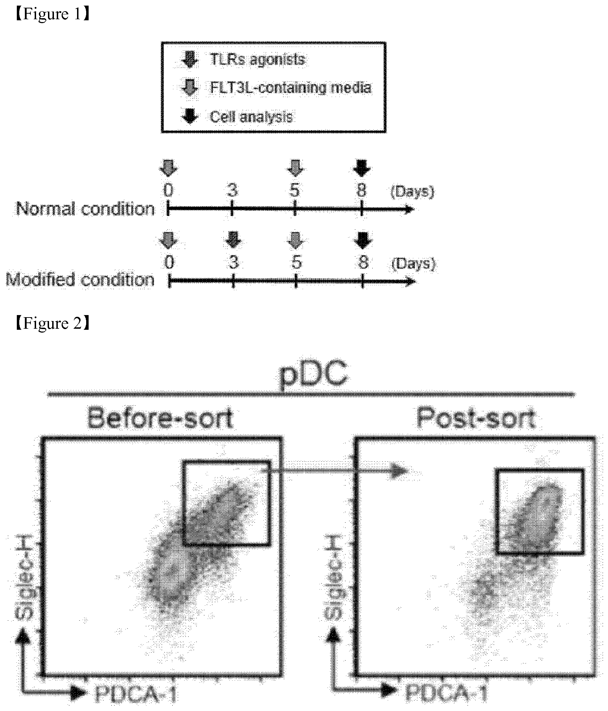

FIG. 1 shows a schematic design diagram of a treatment method of the immature dendritic cells using a differentiation-inducing factor (Flt3L-containing media) and toll-like receptor agonist (TLRs agonists) according to one embodiment of the present disclosure.
FIG. 2 shows separation of plasmacytoid dendritic cells (TLRs-pDC) as differentiated in an induced manner after treatment using the toll-like receptor agonist (Pam3) according to one embodiment of the present disclosure in Example 1.

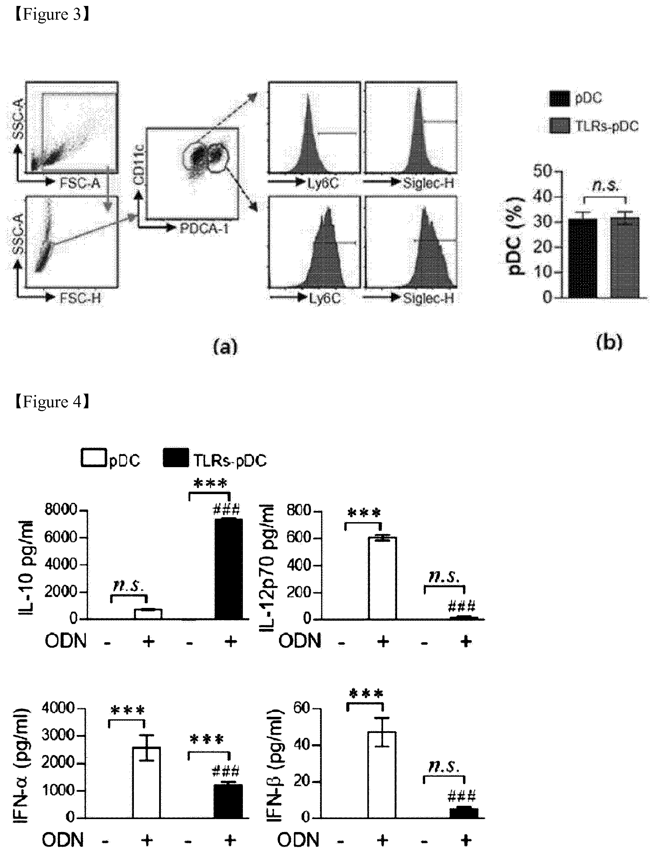

FIG. 3A shows surface expression molecules specifically induced to the plasmacytoid dendritic cells as differentiated in an induced manner after treatment using the toll-like receptor agonist (Pam3) according to one embodiment of the present disclosure in Example 1.
FIG. 3B shows a ratio of the number of plasmacytoid dendritic cells as differentiated in an induced manner after treatment using the toll-like receptor agonist (Pam3) according to one embodiment of the present disclosure, compared to the number of the immature dendritic cells in Example 1.
FIG. 4 is a graph showing comparison between expression patterns of cytokine (IFN-α, IFN-β, IL-12p70, and IL-10) from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 2.

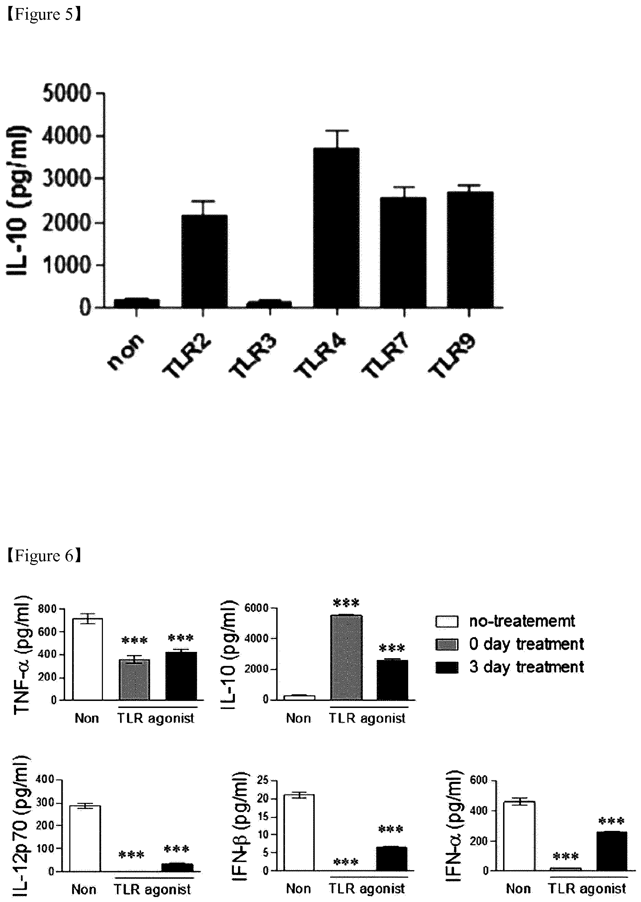

FIG. 5 is a graph showing comparison between expression patterns of cytokine (IL-10) from the plasmacytoid dendritic cells as differentiated in an induced manner after treatment using various toll-like receptor agonists according to one embodiment of the present disclosure and the conventional plasmacytoid dendritic cells (non) in Example 3.
FIG. 6 is a graph showing comparison between expression patterns of cytokine (IFN-α, IFN-β, TNF-α, IL-12p70, and IL-10) based on treatment timing of the immature dendritic cells using the toll-like receptor agonist (Pam3) according to one embodiment of the present disclosure in Example 4.

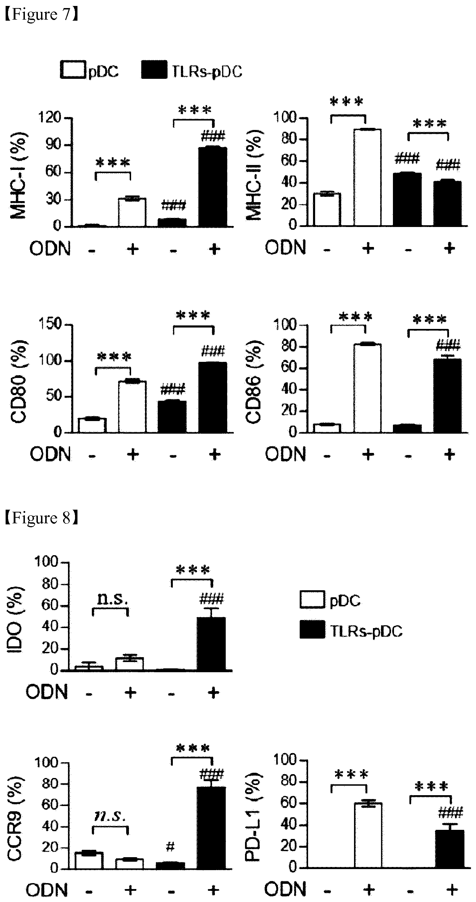

FIG. 7 is a graph showing comparison between expression patterns of MHC complex molecule, CD80, and CD86 from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 5.
FIG. 8 is a graph showing comparison between expression patterns of IDO, CCR9, and PD-L1 from the plasmacytoid dendritic cells (TLRs-pDC) as differentiated in an induced manner after treatment using the toll-like receptor agonist (Pam3) according to one embodiment of the present disclosure and the conventional plasmacytoid dendritic cells (pDC) in Example 6.

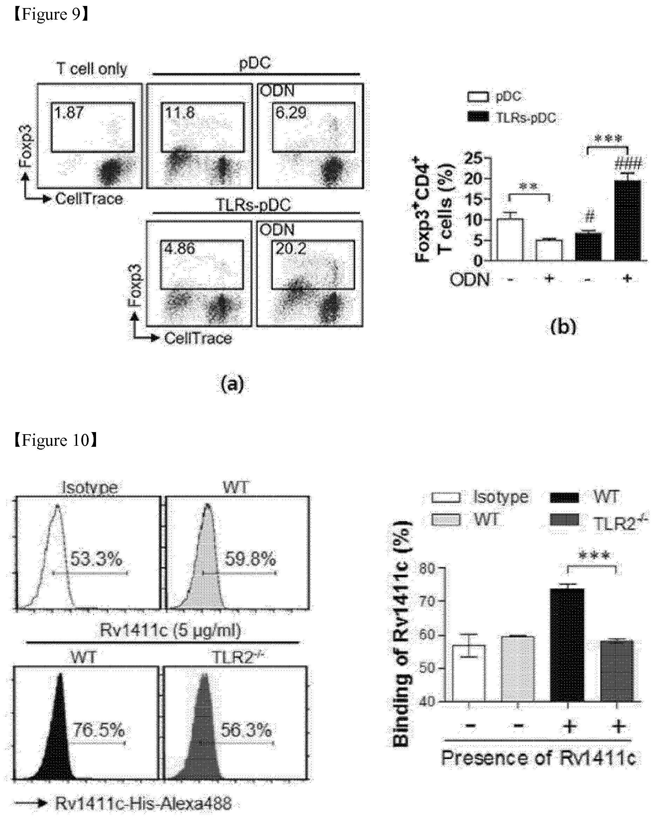

FIG. 9A shows measurement results using a flow cytometer LSRFortessa x-20 of whether proliferation into regulatory T cells is achieved by treatment using the plasmacytoid dendritic cells (TLRs-pDC) as differentiated in an induced manner after treatment using the toll-like receptor agonist (Pam3) according to one embodiment of the present disclosure or using the conventional plasmacytoid dendritic cells (pDC) in Example 7 regarding T cells.
FIG. 9B shows measurement results of a proliferation rate of regulatory T cells by treatment using the plasmacytoid dendritic cells (TLRs-pDC) as differentiated in an induced manner after treatment using the toll-like receptor agonist (Pam3) according to one embodiment of the present disclosure or using the conventional plasmacytoid dendritic cells (pDC) in Example 7 regarding T cells.
FIG. 10 shows the bindings between wild-type dendritic cells (WT) and dendritic cells (TLR2 -/-) isolated from TLR2 knockout mice and Rv1411c protein in Example 8.

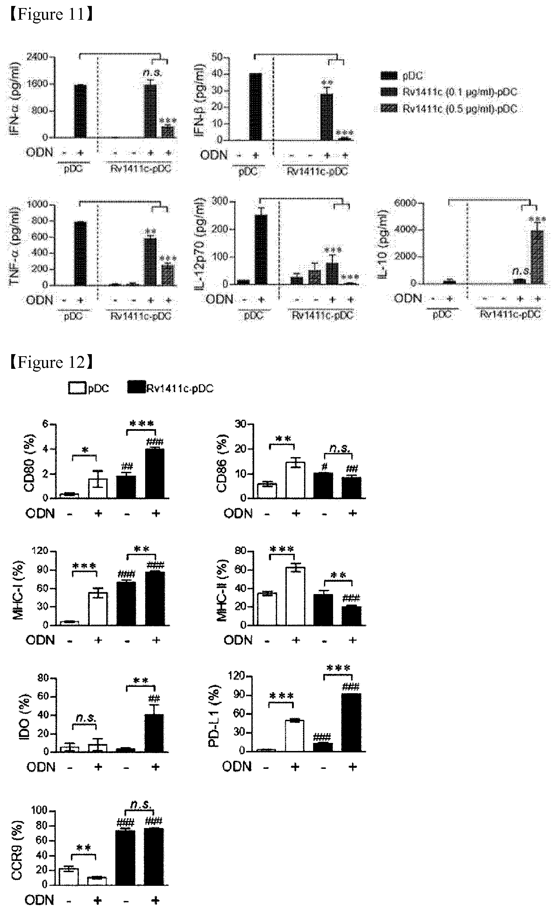

FIG. 11 is a graph showing comparison results between expression patterns of cytokine(IFN-α, IFN-β, TNF-α, IL-12p70, IL-10) from plasmacytoid dendritic cells (Rv1411c(0.1 µg/ml)-pDC, Rv1411c(0.5 µg/ml)-pDC) as differentiated in an induced manner after treatment using a toll-like receptor agonist (Rv1411c protein(0.1µg/ml, 0.5 µg/ml)) according to one embodiment of the present disclosure and from the conventional plasmacytoid dendritic cells (pDC) in Example 9.
FIG. 12 is a graph showing comparison results between expression patterns of MHC complex molecule, CD80, CD86, and immune tolerance inducing molecule (IDO, CCR9 and PD-L1) from the plasmacytoid dendritic cells (Rv1411c-pDC) as differentiated in an induced manner after treatment using the toll-like receptor agonist (Rv1411c protein) according to one embodiment of the present disclosure and the conventional plasmacytoid dendritic cells (pDC) in Example 10.

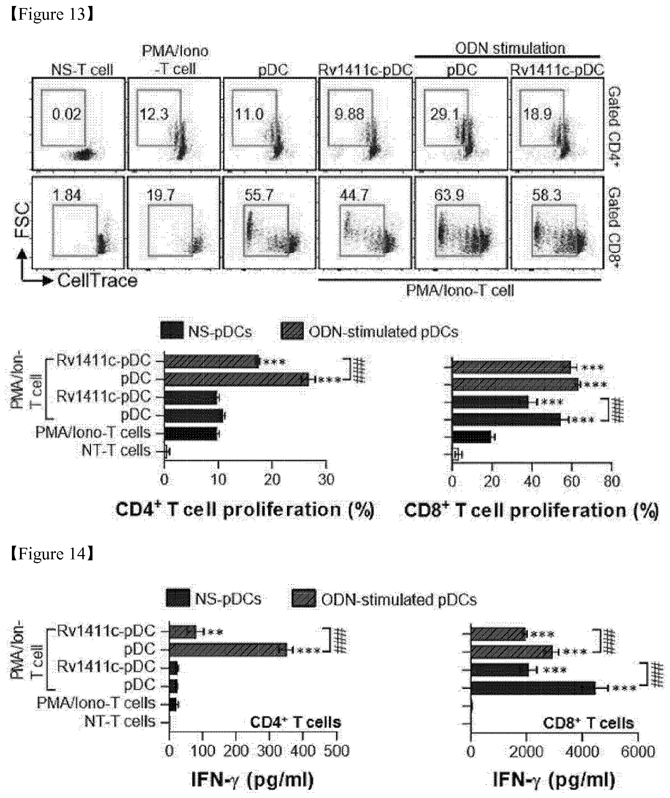

FIG. 13 shows comparison results of T cell proliferation as obtained by mixing and culturing T cells and the plasmacytoid dendritic cells (Rv1411c-pDC) as differentiated in an induced manner after treatment using the toll-like receptor agonist (Rv1411c protein) according to one embodiment of the present disclosure and T cell proliferation as obtained by mixing and culturing T cells and the conventional plasmacytoid dendritic cells (pDC) in Example 11.
FIG. 14 shows comparison results of secretion pattern of IFN-gamma as obtained by mixing and culturing T cells and the plasmacytoid dendritic cells (Rv1411c-pDC) as differentiated in an induced manner after treatment using the toll-like receptor agonist (Rv1411c protein) according to one embodiment of the present disclosure and secretion pattern of IFN-gamma as obtained by mixing and culturing T cells and the conventional plasmacytoid dendritic cells (pDC) in Example 11.

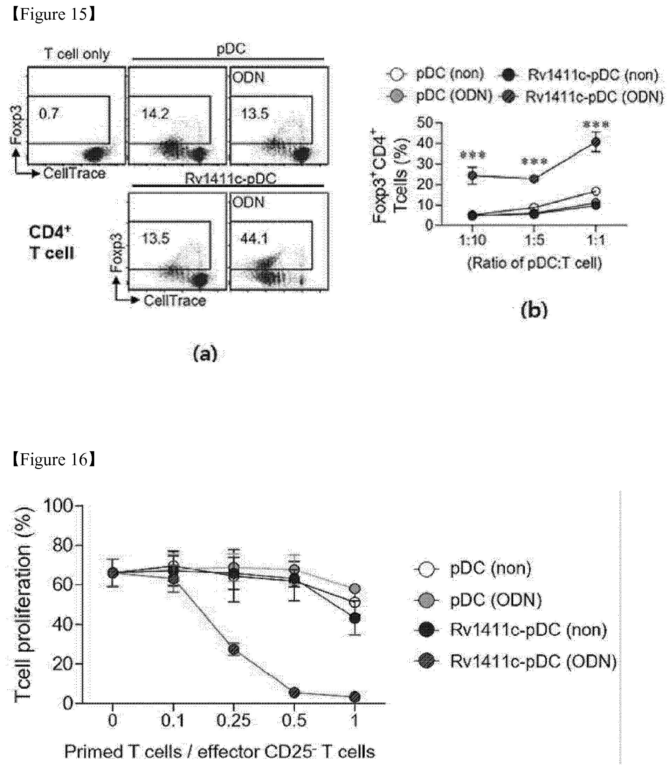

FIG. 15A shows measurement results using a flow cytometer LSRFortessa x-20 of whether proliferation into regulatory T cells is achieved by treatment using the plasmacytoid dendritic cells (Rv1411c-pDC) as differentiated in an induced manner after treatment using the toll-like receptor agonist (Rv1411c protein) according to one embodiment of the present disclosure or using the conventional plasmacytoid dendritic cells (pDC) in Example 12 regarding T cells.
FIG. 15B shows measurement results of a proliferation rate of regulatory T cells by treatment using the plasmacytoid dendritic cells (Rv1411c-pDC) as differentiated in an induced manner after treatment using the toll-like receptor agonist (Rv1411c protein) according to one embodiment of the present disclosure or using the conventional plasmacytoid dendritic cells (pDC) in Example 12 regarding T cells.
FIG. 16 is a graph showing the results of measurement of changes in T cell proliferation ability based on a ratio of primed T cells to CD25-effector T cells, as obtained after culturing, together with CD4 + and CD25-effector T cells, T cells as differentiated by the plasmacytoid dendritic cells (RVc1411c-pDC) as differentiated in an induced manner after treatment using the toll-like receptor agonist (Rv1411c protein) according to one embodiment of the present disclosure in Example 13.

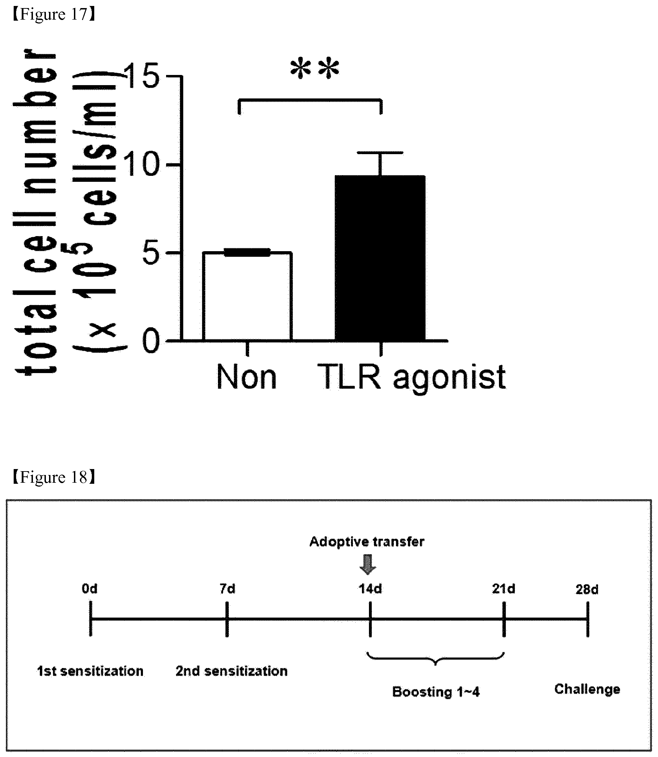

FIG. 17 shows the comparison results between the number of plasmacytoid dendritic cells (TLR agonist) induced after treatment with toll-like receptor agonist (Rv1411c protein and Pam3) according to one embodiment of the present disclosure and the number of the plasmacytoid dendritic cells induced after non-treatment (Non) using the toll-like receptor agonist in Example 14.
FIG. 18 shows an experimental design for constructing an ovalbumin (OVA)-derived food allergic disease animal model in Example 15.

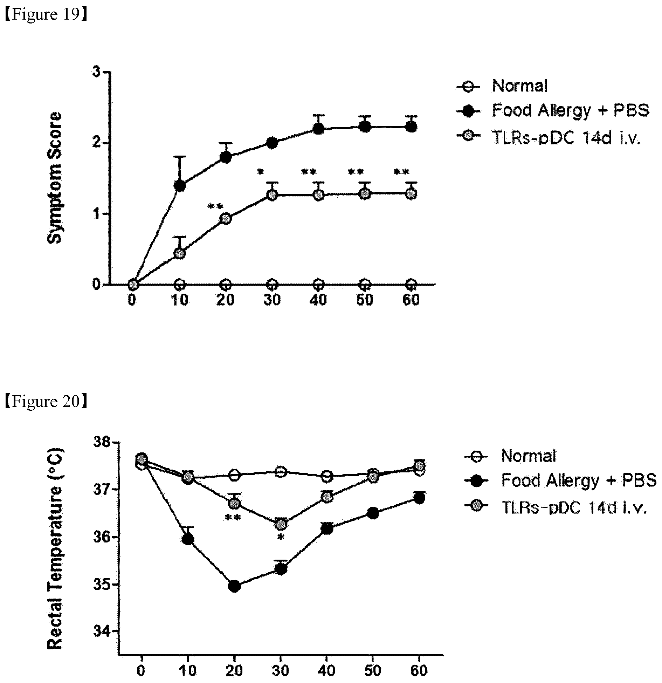

FIG. 19 is a graph showing the evaluation results of systemic anaphylactic symptom after the plasmacytoid dendritic cells (TLRs-pDC 14d i.v.) differentiated in an induced manner after application of a toll-like receptor agonist (Rv1411c protein) according to one embodiment of the present disclosure was applied to an ovalbumin (OVA)-derived food allergic disease animal model in Example 15.
FIG. 20 is a graph showing the results of measurement of body temperature change in the rectum after the plasmacytoid dendritic cells (TLRs-pDC 14d i.v.) differentiated in an induced manner after application of a toll-like receptor agonist (Rv1411c protein) according to one embodiment of the present disclosure was applied to an ovalbumin (OVA)-derived food allergic disease animal model in Example 15.

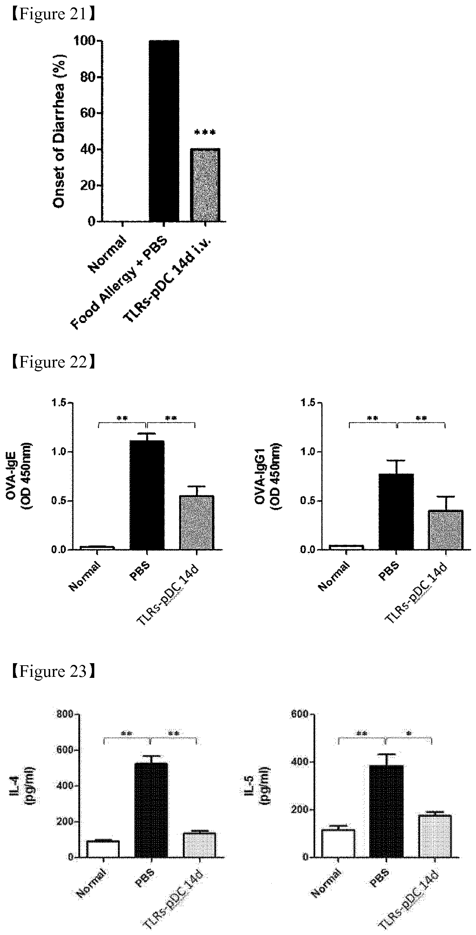

FIG. 21 is a graph showing the results of measuring occurrence of diarrhea after the plasmacytoid dendritic cells (TLRs-pDC 14d i.v.) differentiated in an induced manner after application of a toll-like receptor agonist (Rv1411c protein) according to one embodiment of the present disclosure was applied to an ovalbumin (OVA)-derived food allergic disease animal model in Example 15.
FIG. 22 is a graph showing the results of in-blood IgE and IgG1 concentration measurements after the plasmacytoid dendritic cells (TLRs-pDC 14d) differentiated in an induced manner after application of a toll-like receptor agonist (Rv1411c protein) according to one embodiment of the present disclosure was applied to an ovalbumin (OVA)-derived food allergic disease animal model in Example 16.
FIG. 23 is a graph showing the results of measurement of the concentrations of in-blood interleukin-4 (IL-4) and interleukin-5 (IL-5) after the plasmacytoid dendritic cells (TLRs-pDC 14d) differentiated in an induced manner after application of a toll-like receptor agonist (Rv1411c protein) according to one embodiment of the present disclosure was applied to an ovalbumin (OVA)-derived food allergic disease animal model in Example 17.

In FIG. 1 to FIG. 23, * means P < 0.05, ** means P < 0.01 and *** means P < 0.005.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented herein.

The present disclosure relates to a production method of a pharmaceutical composition for prevention or treatment of hypersensitivity immune diseases, the method including the step of producing following tolerogenic plasmacytoid dendritic cells.

In accordance with the present disclosure, producing the aforementioned tolerogenic plasmacytoid dendritic cells may include treating the dendritic cells with toll-like receptor agonists.

As used herein, the term "dendritic cells (DCs)" refers to a professional antigen presenting cell for absorbing the antigen into the cell and presenting various antigenic samples to the T cell together with MHC (major histocompatibility complex) class I complex or MHC class II complex. Further, the dendritic cells may include both immunogenic and tolerogenic antigen presenting cells, and may be classified into the immature dendritic cells ("imDC"), semimature dendritic cells ("smDC") and mature dendritic cells ("mDC") according to their maturity.

As used herein, the term, "immature dendritic cells" is found in the early mature stages of the dendritic cells. The immature dendritic cell does not express CD14 as the surface phenotype of the monocyte cell. The immature dendritic cell may express one of the co-stimulatory molecules CD40, CD54, CD80, CD86 and CD274 at a lower level than the mature dendritic cells may express.

As used herein, the term, "semimature dendritic cells" refers to dendritic cells that lose some of the properties of the immature dendritic cells, and have some characteristics of the phenotype of mature dendritic cells. This semimature dendritic cell may mean a dendritic cell that exhibits partially or incompletely mature form and phenotypic characteristics.

As used herein, the term, "mature dendritic cells" refers to cells into which the immature dendritic cells are matured. The mature dendritic cells express high levels of MHC class II, CD40, CD54, CD80, CD86 and CD274 as well as DC-LAMP, and release anti-inflammatory cytokines. The mature dendritic cells may be characterized by the ability to cause an increase in proliferation of primitive allogeneic T cells and syngeneic T cells and/or an increased production of dendritic cells cytokines in a mixed lymphocyte reaction. The mature dendritic cells typically express high levels of CCR7 and CXCR4.

However, in accordance with the present disclosure, the dendritic cells to be treated with the toll-like receptor agonist are preferably the immature dendritic cells that have not undergone differentiation. In this connection, the immature dendritic cells may include naive dendritic cells and may be isolated and obtained from the mammalian bone marrow and the like.

Further, in accordance with the present disclosure, the tolerogenic dendritic cells differentiated in the induced manner from the immature dendritic cells by treating the immature dendritic cells using the toll-like receptor agonist may be tolerogenic plasmacytoid dendritic cells (pDC).

As used herein, the term, "plasmacytoid dendritic cells" is a subset of dendritic cells. The plasmacytoid dendritic cell was first known by histologically finding the shape of a plasma cell in a human lymph node by Dr. Lennert and Dr. Remmele in 1958. It is known that the plasmacytoid dendritic cells do not express B cell specific marker (immunoglobulin). Thus, the plasmacytoid dendritic cells are named as plasmacytoid T cells. It is known that the plasmacytoid dendritic cells express myeloid lineage antigen and MHC class II while not expressing CD3 which is a common marker of the T cell. Thus, the plasmacytoid dendritic cell is named as plasmacytoid monocyte. Thereafter, it is known that the plasmacytoid dendritic cell has the ability to induce an allergenic mixed lymphocyte reaction (MLR), which is an intrinsic property of dendritic cells. Thus, the plasmacytoid dendritic cell is called plasmacytoid dendritic cells, simply, as 'pDC'.

As used herein, the term "tolerogenic" refers not only to a state that the cell does not exhibit an immune response to a specific antigen, but also to a state that the cell inhibits the immune response. Therefore, in accordance with the present disclosure, the "tolerogenic plasmacytoid dendritic cells" promote secretion of anti-inflammatory cytokines such as IL-10, and inhibit the secretion of inflammatory cytokines such as IL-12p70 and TNF-α. Recently, the tolerogenic plasmacytoid dendritic cells express indoleamine-2,3 dioxygenase (IDO) known as immune tolerance inducing molecules, and CCR9 as a surface molecule of the immune tolerance inducing cells at high levels, thereby to induce differentiation of regulatory T cells and to inhibit the activity of effector T cells.

In accordance with the present disclosure, the tolerogenic plasmacytoid dendritic cells may be produced stably and in large quantities by treating the immature dendritic cells using the toll-like receptor agonist before differentiation initiation or during the differentiation of the immature dendritic cells. In this connection, the "before differentiation initiation" may include the time before the differentiation-inducing factor is applied to the immature dendritic cells. Further, the duration "during differentiation" refers to the duration from a time when the differentiation-inducing factor is applied to the immature dendritic cells to a time before the differentiation is completed by the differentiation-inducing factor. Preferably, the duration during differentiation may include a duration from the time of the applying of the differentiation-inducing factor to 7 days after the treatment. However, the present disclosure is not limited thereto.

In accordance with the present disclosure, the application timing of the toll-like receptor agonist is not limited to a specific time point as long as the toll-like receptor agonist is applied prior to or during differentiation of the immature dendritic cells as described above. In accordance with the present disclosure, when the toll-like receptor agonist is applied thereto during the differentiation of the immature dendritic cells, the toll-like receptor agonist may be applied thereto within 7 days (168 hours), 5 days (120 hours) or 3 days (72 hours) from the differentiation initiation. However, the present disclosure is not limited thereto. Further, in accordance with the present disclosure, when the toll-like receptor agonist is applied thereto prior to the initiation of differentiation of the immature dendritic cells, the differentiation-inducing factors may be used to initiate the differentiation of the immature dendritic cells within 36 hours, preferably 24 hours, from the application of the toll-like receptor agonist. However, the present disclosure is not limited thereto.

Further, in accordance with the present disclosure, the toll-like receptor agonist may be applied once or more than once. The specific application times are not particularly limited. In one example, when applying the toll-like receptor agonist thereto during the differentiation of the immature dendritic cells, the toll-like receptor agonist may be applied thereto at least once within 7 days, 5 days or 3 days from the start of differentiation of the immature dendritic cells. Further, when applying the toll-like receptor agonist prior to the initiation of differentiation of the immature dendritic cells, the differentiation of the immature dendritic cells may be initiated within 36 or 24 hours from the treatment time point after the treatment of the immature dendritic cells with the toll-like receptor agonist at least once, and then, one or more additional treatments may optionally be carried out during the differentiation.

In this connection, the "differentiation initiation" refers to the addition of the differentiation-inducing factor to the culture medium of the immature dendritic cells, or to the inoculation of the immature dendritic cells into a medium containing the differentiation-inducing factors. However, the differentiation initiation is not particularly limited as long as the differentiation initiation may induce the differentiation of the immature dendritic cells using the differentiation-inducing factor.

As used herein, the term, "toll-like receptor agonist" may refer to a conserved molecular substance derived from a pathogen and may be pathogen associated molecular patterns (PAMPs). In this connection, the pathogen may be gram-positive bacteria, gram-negative bacteria, fungi or viruses. Further, the toll-like receptor agonist may be endogenous molecules released from damaged or dead cells and may be DAMPs (Damage Associated Molecular Patterns). DAMPs or PAMPs initiate an immune response via the TLR signal and collect adapter molecules within cytoplasm of the cell to deliver the signal. The toll-like receptor agonist may be fragments, variants, analogs, homologs or derivatives of PAMPs or DAMPs that bind to the toll-like receptors, and induce TLR-mediated activation, such as activation of NF-kB. The fragments, variants, analogs, homologs or derivatives as the toll-like receptor agonist are at least 30 to 99% identical to the amino acids of the TLR agonist and induce activation resulting from toll-like receptor-mediated activation.

The type of toll-like receptor agonist applied to the immature dendritic cells in accordance with the present disclosure is not particularly limited but, preferably, is a PAMPs ligand. More preferably, the type of toll-like receptor agonist applied to the immature dendritic cells is capable of inducing the differentiation of the tolerogenic plasmacytoid dendritic cells from the immature dendritic cells via MyD88 (Myeloid differentiation primary response gene 88) signal.

Further, in accordance with the present disclosure, the toll-like receptor agonist includes at least one selected from the group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist. More specifically, one or more of the ligands listed in Table 1 below may be included in the toll-like receptor agonist. However, the present disclosure is not particularly limited thereto. As long as any ligand can be bound to the toll-like receptor of the dendritic cell and may encounter the MyD88 signal, any ligand may be used without limitation.

Further, in accordance with the present disclosure, when the TLR2 agonist is used as the toll-like receptor agonist, one or more of the TLR1 and TLR6 agonists acting as the co-receptor for the TLR2 agonist may be further included in the toll-like receptor agonist. In this connection, specific examples of the TLR1 agonist and TLR6 agonist may be the ligands listed in Table 1 below. The ligands as the TLR1 agonist and TLR6 agonist may not be particularly limited as long as the ligands are capable of acting as a co-receptor for the TLR2 agonist.

**[Table 1]**

| Ligand | PAMPs(Pathogen Associated Ligands) | Ligand natural host | Synthetic ligand |
|---|---|---|---|
| TLR1 | Multiple triacyl lipopeptides | Bacteria | Pam3Cys-* |
| TLR2 | Multiple glycolipids | Bacteria | CFA |
| | Multiple lipopeptides | Bacteria | Pam3, MALP2-** |
| | Multiple lipoproteins | Bacteria | Pam2Cys** |
| | Lipoteichoic acid | Gram-positive bacteria | FSL-1 |
| | HSP 70, or other heat shock proteins | Host cells | Hib-OMPC |
| | Zymosan (Beta- glucan) | Fungi | |
| | Protein | Mycobacteriu m tuberculosis | Rv1411c protein, ESAT-6, PE/PPE Protein, Rv0577 |
| TLR3 | Double stranded RNA | Virus | Poly (I : C); Low molecular weight or high molecular weight Poly (A : U) |
| TLR4 | Lipopolysaccharides (LPS); or IPS analog (MPLA) | Gram negative bacteria | AGP |
| | Heat shock protein | Bacteria and host cell | MPLA |
| | Fibrinogen | Host cell | RC-529 |
| | Heparin sulfate fragments | Host cell | MDF2B |
| | Hyaluronic acid fragments | Host cell | CFA |
| | Nickel | | |
| | Various opoid drugs | | |
| | Protein | Mycobacteriu m tuberculosis | RpfE, RpfB, Rv2299c, HBHA |
| TLR5 | Flagellin | Bacteria | Flagellin |
| TLR6 | Multiple diacyl lipopeptides | Mycoplasma | FSL1-** |
| | | | Pam2Cys** |
| | | | MALP2-** |
| TLR7 | Virus ssRNA(influenza, VSV, HIV, HCV) | RNA virus | Guanosine analogs; imidazoquinolines (e.g. Imiquimod, Aldara ® R848, Resiquimod)®), loxoribine |
| TLR8 | Small synthetic compounds; single-stranded RNA | RNA, human and virus | Imidazoquinolines; loxoribine; ssPolyU, 3M-012 |
| TLR9 | Unmethylated CpG Oligodeoxynucleotide DNA; DNA; dsDNA virus(HSV, MCMV); Hemozoin(Plasmodium) | Bacteria, DNA virus | CpG-oligonucleotides, various sequences as synthesized (e.g. CpG-ODN 2006, 1826,2395) |
| TLR10 | | | |
| TLR11 | Profilin | Toxoplasma gondii | |
| TLR12 | Profilin | Toxoplasma gondii | |
| TLR13 | Bacterial ribosomal RNA sequence "CGGAAAGACC" | Virus, bacteria | |
| * Ligands recognized by TLR1 and TLR2 | | | |
| ** Ligands recognized by TLR2 and TLR6 | | | |

In accordance with the present disclosure, the toll-like receptor agonist may be derived from microorganisms, viruses, plants or animals, or may be synthesized. There is no particular restriction on a source thereof.

Further, the differentiation of the immature dendritic cells in accordance with the present disclosure may be performed using the differentiation-inducing factor. In this connection, it is preferable to use the FMS-like tyrosine kinase 3 ligand (Flt3L) as the differentiation-inducing factor. The differentiation-inducing factor is not particularly limited as long as the differentiation-inducing factor can induce the differentiation of the immature dendritic cells into plasmacytoid dendritic cells and conventional dendritic cells (conventional plasmacytoid cells, cDCs).

Further, in accordance with the present disclosure, the differentiation of the immature dendritic cells may be accomplished by incubating the immature dendritic cells in a medium containing the differentiation-inducing factors, by adding the differentiation-inducing factor to the culture medium for the immature dendritic cells.

Further, in accordance with the present disclosure, optionally, the differentiation-inducing factor may be added one or more times during the differentiation of the immature dendritic cells.

As used herein, the term, "FMS-like tyrosine kinase 3 ligand (Flt3L)" refers to an endogenous small molecule that acts as a cytokine and growth factor by activating hematopoietic progenitors.

Further, in accordance with the present disclosure, the duration of differentiation of the immature dendritic cells is not particularly limited. The duration of differentiation of the immature dendritic cells may vary depending on the type of medium to be used and the environment. For example, the duration of differentiation of the immature dendritic cells may be in range from 5 days to 15 days, preferably from 7 days to 10 days, more preferably from 8 days to 9 days.

In accordance with the present disclosure, immune tolerance of the plasmacytoid dendritic cells may be activated by further applying the toll-like receptor agonist to the tolerogenic plasmacytoid dendritic cells differentiated in an induced manner from the immature dendritic cells by treating the immature dendritic cells using the toll-like receptor agonist.

In accordance with the present disclosure, the types of toll-like receptor agonist used to activate the tolerogenic plasmacytoid dendritic cells differentiated as described above are not particularly limited but may include at least one selected from the group consisting of a TLR1 agonist, a TLR2 agonist, a TLR3 agonist, a TLR4 agonist, a TLR5 agonist, a TLR6 agonist, a TLR7 agonist, a TLR8 agonist, a TLR9 agonist, a TLR11 agonist, a TLR12 agonist, and a TLR13 agonist. Preferably, the TLR9 agonist may be used.

In accordance with the present disclosure, the differentiation of the immature dendritic cells into the tolerogenic plasmacytoid dendritic cells may be effectively induced through the above process. Therefore, it is possible to stably supply large amount of the tolerogenic plasmacytoid dendritic cells via the simple and easy process using the immature dendritic cells.

Further, in accordance with the present disclosure, the tolerogenic plasmacytoid dendritic cells obtained as described above inhibit the expression of inflammatory cytokines, and promote expression of anti-inflammatory cytokines, and induce differentiation of regulatory T cells and inhibit the activity of effector T cells, thereby effectively suppressing the immune response. Furthermore, the expression of allergen-specific immunoglobulins and Th2 cytokines can be inhibited by the tolerogenic plasmacytoid dendritic cells obtained as described above to effectively prevent or treat various hypersensitivity immune diseases including food-derived allergic diseases.

As used herein, the term "hypersensitivity immune diseases" refers to an excessive immune response to one or more antigen exposure times. For example, when substances that do not cause any problems in humans, such as pollen, may come into contact with the skin or be digested therein, the immune system of the human body causes problems in term of metabolism and lesion in the body. The hypersensitivity immune diseases refer to any disease that is caused by an excessive immune response via the memory cell when the body repeatedly contacts an external substance and is harmful to the human body.

Specifically, as used herein, the hypersensitivity immune disease may be selected from allergic urticaria, allergic rhinitis, allergic conjunctivitis, allergic asthma, allergic dermatitis, autoimmune hepatitis, allergic bronchopulmonary aspergillosis, and allergic stomatitis. However, the present disclosure is not limited thereto.

As used herein, the term "prevention" refers to any act that inhibits or delays progression of hypersensitivity immune diseases by administration of the composition in accordance with the present disclosure.

As used herein, the term "treatment" and "amelioration" refer to any act that ameliorates or changes the symptoms of hypersensitivity immune diseases in a positive manner by administration of the composition in accordance with the present disclosure.

According to the present disclosure, the pharmaceutical composition may be characterized as being in the form of capsules, tablets, granules, injections, ointments, powders or beverages. The pharmaceutical composition may be characterized by targeting to humans.

The pharmaceutical composition in accordance with the present disclosure may be formulated in the form of oral preparations such as powders, granules, capsules, tablets, aqueous suspensions, external preparations, suppositories and sterilized injection solutions according to a conventional method. The present disclosure is not limited thereto. The pharmaceutical composition in accordance with the present disclosure may include pharmaceutically acceptable carriers. The pharmaceutically acceptable carrier may include a binder, a lubricant, a disintegrant, an excipient, a solubilizing agent, a dispersing agent, a stabilizer, a suspending agent, a pigment, a perfume, etc., upon oral administration. In the case of an injectable preparation, the pharmaceutically acceptable carrier may include a buffer, a preservative, an anhydrous agent, a solubilizer, an isotonic agent, a stabilizer, etc. In the case of topical administration, the pharmaceutically acceptable carrier may include a base, an excipient, a lubricant, a preservative, etc. Formulations of the pharmaceutical composition in accordance with the present disclosure may be produced in a variety of ways, mixed with a pharmaceutically acceptable carrier as described above. For example, when administered orally, it may be produced in the form of tablets, troches, capsules, elixirs, suspensions, syrups, wafers, etc. In the case of injections, it may be produced in a unit dose ampoule or multiple doses. It may be produced in the forms of other solutions, suspensions, tablets, capsules, sustained-release preparations and the like.

Further, examples of carriers, excipients and diluents suitable for formulation include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, or the like. Further, fillers, anti-coagulants, lubricants, wetting agents, perfumes, emulsifiers, preservatives and the like may be further included.

The administration route of the pharmaceutical composition according to the present disclosure is not limited to following but includes oral, intravenous, intramuscular, intraarterial, intramedullary, intradural, intracardiac, transdermal, subcutaneous, intraperitoneal, intranasal, intestinal, topical, sublingual or rectal. Oral or parenteral administration is preferred.

As used herein, "parenteral" includes subcutaneous, intradermal, intravenous, intramuscular, intraarticular, intrasynovial, intrasternal, intrathecal, intralesional and intracranial injection or infusion techniques. The pharmaceutical composition in accordance with the present disclosure may be further administered in the form of suppositories for rectal administration.

The pharmaceutical composition in accordance with the present disclosure can vary widely depending on various factors including the activity of the specific compound used, age, body weight, and conventional health, gender, formula, administration time, administration route, excretion rate, drug combination and prevention or severity of specific disease to be treated. The amount of administration of the pharmaceutical composition will depend on the patient's condition, weight, severity of disease, drug form, administration route and duration, but may be appropriately selected by one skilled in the art and may be injected at a content of 0.0001 to 50 mg/kg or 0.001 to 50 mg/kg per day. Administration may be done once a day, or several sessions a day. The amount of administration does not in any way limit the scope of the present disclosure. The pharmaceutical composition according to the present disclosure may be formulated into pills, caplets, capsules, liquids, gels, syrups, slurries, and suspensions.

The composition in accordance with the present disclosure may be used alone. Alternatively, it may be used in combination with methods that use surgery, radiation treatment, hormone treatment, chemical treatment, and biological response modifiers.

Further, the present disclosure relates to a method of treating hypersensitivity immune diseases, including the step of administering the pharmaceutical composition according to the present disclosure in a pharmaceutically effective amount to an entity having hypersensitivity immune diseases.

Further, the present disclosure relates to the prevention method of hypersensitivity immune diseases, including the step of administering the pharmaceutical composition according to the present disclosure in a pharmaceutically effective amount to an entity having hypersensitivity immune diseases.

The pharmaceutical composition administered in the treatment method or the prevention method of the present disclosure may be the same as described above. In order to avoid the excessive complexity of the description, the description thereof is omitted.

In accordance with the present disclosure, the pharmaceutical composition can be administered either orally or parenterally. Parenteral administration may be performed by intraperitoneal, intrarectal, subcutaneous, intravenous, intramuscular, or intrauterine dural injection, intracerebroventricular injection or intrathoracic injection. Further, the composition may be administered orally in clinical administration and can be used in the form of conventional pharmaceutical preparations.

In accordance with the present disclosure, the number of administrations of the pharmaceutical composition is not particularly limited. The administration may be performed at least once. More specifically, depending on the purpose of prevention and treatment, the administration may be performed one or more times. However, when the symptom is present, the administration may be repetitive.

In this connection, the pharmaceutically effective amount may be in a range of 0.0001 to 50 mg/kg or 0.001 to 50 mg/kg. However, the present is not limited thereto. The amount of administration can vary depending on the specific patient's weight, age, sex, health status, diet, administration period, administration method, clearance rate, severity of disease, and the like.

As used herein, the term, "administration" means providing a composition in accordance with the present disclosure to an entity in any appropriate manner.

As used herein, the term "entity" refers to all animals, including humans, monkeys, dogs, goats, pigs, or rats, who have the hypersensitivity immune diseases which may be cured by administering the composition in accordance with the present disclosure thereto.

As used herein, the term, "pharmaceutically effective amount" means an amount sufficient to cure the disease at a reasonable benefit or risk applicable to medical treatment. This may be determined by factors well known in the medical field such as the type of disease, severity of the entity, drug activity, drug sensitivity, administration time, administration route and rate of exposure, duration of treatment, etc.

Further, the present disclosure relates to the production method of a cosmetic composition for the prevention or amelioration of hypersensitivity immune diseases, the method including the step of producing the tolerogenic plasmacytoid dendritic cells.

In accordance with the present disclosure, a detailed description of a method for inducing the tolerogenic plasmacytoid dendritic cells by applying the toll-like receptor agonist to immature dendritic cells may be the same as described with reference to the pharmaceutical composition. In order to avoid the excessive complexity of the description, the description thereof is omitted.

In accordance with the present disclosure, the cosmetic composition may be produced in form of lotion, nutrition lotion, nutrition essence, massage cream, beauty bath additive, body lotion, body milk, bath oil, baby oil, baby powder, shower gel, shower cream, sunscreen lotion, sunscreen cream, suntan cream, skin lotion, skin cream, sunscreen cosmetics, cleansing milks, depilatory products (for cosmetics), face and body lotions, face and body creams, skin whitening cream, hand lotion, hair lotion, cosmetic cream, jasmine oil, bath soap, water soap, beauty soap, shampoo, hand cleaner, medicinal soap (non-medical use), cream soap, facial wash, whole body cleanser, scalp cleanser, hair rinse, cosmetic soap, tooth whitening gel, toothpaste, etc. To this end, the composition in accordance with the present disclosure may further include solvents commonly used in the production of cosmetic compositions, or suitable carriers, excipients or diluents.

The type of the solvent that may be added to the cosmetic composition in accordance with the present disclosure is not particularly limited. For example, water, saline, DMSO or a combination thereof may be used as the solvent. Examples of carriers, excipients or diluents include purified water, oils, waxes, fatty acids, fatty acid alcohols, fatty acid esters, surfactants, humectants, thickeners, antioxidants, viscosity stabilizers, chelating agents, buffering agents, lower alcohols, and the like. However, the present disclosure is not limited thereto. Further, whitening agents, moisturizers, vitamins, sunscreens, perfumes, dyes, antibiotics, antibacterial agents, and antifungal agents may be included as needed.

As the oil, hydrogenated vegetable oil, castor oil, cottonseed oil, olive oil, palm oil, jojoba oil, and avocado oil may be used. As the wax, beeswax, wax, carnauba, candelilla, montan, ceresin, liquid paraffin, and lanolin may be used.

As the fatty acid, stearic acid, linoleic acid, linolenic acid and oleic acid may be used. Examples of the fatty acid alcohol may include cetyl alcohol, octyldodecanol, oleyl alcohol, panthenol, lanolin alcohol, stearyl alcohol, hexadecanol. As fatty acid esters, isopropyl myristate, isopropyl palmitate, and butyl stearate may be used. As the surfactant, cationic surfactants, anionic surfactants and nonionic surfactants known in the art may be used. Surfactants derived from natural products may be preferred.

In addition, the cosmetic composition may include hygroscopic agents, thickeners, antioxidants, etc. well known in the field of cosmetics. The types and amounts thereof are as well known in the art.

Further, the present disclosure relates to the production method of a food composition for prevention or amelioration of hypersensitivity immune diseases, the method including the step of producing the tolerogenic plasmacytoid dendritic cells.

In the present disclosure, a detailed description of the method for inducing tolerogenic plasma cytosolic dendritic cells by applying a toll-like receptor agonist to immature dendritic cells may be the same as described with reference to the pharmaceutical composition. In order to avoid the excessive complexity of the description, the description thereof is omitted.

The food composition in accordance with the present disclosure may be produced in the form of various foods such as beverages, gums, tea, vitamin complexes, powders, granules, tablets, capsules, confectionery, rice cakes, breads, and the like. Because the food composition in accordance with the present disclosure is composed of plant extracts with little toxicity and side effects, the food composition may be safely used for long-term use for prevention purposes.

In accordance with the present disclosure, when the micro-vesicle is included in the food composition, the amount thereof as added thereto may be in a proportion of 0.1 to 50% of the total weight.

In this connection, when the food composition is produced in a beverage form, there are no particular limitations other than a configuration that the beverage contains the food composition in the intended proportion. Various flavors or natural carbohydrates may be added thereto as an additional ingredient as in ordinary beverages. That is, natural carbohydrates include monosaccharides such as glucose, disaccharides such as fructose, polysaccharides such as sucrose, general sugar such as dextrin, and cyclodextrin, and sugar alcohols such as xylitol, sorbitol, and erythritol. Examples of the flavoring agents include natural flavoring agents (such as tau martin and stevia extract (for example, rebaudioside A, glycyrrhizin, and the like)) and synthetic flavorings (for example, saccharin, aspartame, and the like).

In addition, the food composition in accordance with the present disclosure may include various nutrients, vitamins, minerals (electrolytes), flavors such as synthetic flavors and natural flavors, colorants, pectic acid and its salts, alginic acid and its salts, organic acids, protective colloid thickening agents, pH adjusting agents, stabilizers, preservatives, glycerin, alcohols, carbonating agents used in carbonated drinks, and the like.

These components may be used independently or in combination. The proportions of such additives may not be so critical but may be generally selected from the range of from 0.1 to about 50 parts by weight per 100 parts by weight of the food composition in accordance with the present disclosure.

Hereinafter, the present disclosure will be described in more detail with refence to Examples. It should be understood by those skilled in the art that these Example are only for describing the present disclosure more specifically and that the scope of the present disclosure is not limited to these Examples and is in accordance with the gist of the present disclosure.

### Examples

### [Example 1] Regulatory effect of differentiation into plasmacytoid dendritic cells by toll-like receptor agonist

In the treatment of the immature dendritic cells using the toll-like receptor agonists during differentiation of the immature dendritic cells, in order to check whether the immature dendritic cells are differentiated in an induced manner into the tolerogenic plasmacytoid dendritic cells, the following experiment was carried out according to the design diagram shown in FIG. 1.

Specifically, thigh bone marrow samples were collected from C57BL/6 mice using bone marrow collecting syringes. The collected bone marrow was washed with phosphate buffered saline (PBS), and red blood cells were removed therefrom using ammonium chloride. Separated cells (3 × 10⁶ cells/well) were inoculated in a 6-well plate. Then, 1 ml of RPMI 1640 containing 10% FBS (fetal bovine serum), 2 mM L-glutamine, 100 U/ml penicillin/streptomycin, 50 µM mercaptoethanol, 0.1 mM non-essential amino acid, 1 mM sodium pyruvate and 250 ng/ml FLT3L was added thereto to initiate culture and differentiation of the cells. On the third day after the start of differentiation, Pam3 was applied thereto at a concentration of 100 to 500 ng/ml. On 5 days from the start of differentiation, 1 ml of the RPMI 1640 was further supplemented thereto. The cells were cultured for 8 days. From the cells obtained after the incubation, plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA) (FIG. 2). Because the treatment using the toll-like receptor agonist may affect the differentiation into plasmacytoid dendritic cells, surface expression molecules specifically induced from the plasmacytoid dendritic cells were checked on eighth day and the results are shown in FIG. 3A. Further, in the case (TLRs-pDC) when the immature dendritic cells were treated with the toll-like receptor agonist, the ratio of the number of plasmacytoid dendritic cells obtained after the eighth day to the number of the initial immature dendritic cells was measured. The results are shown in FIG. 3B. In this connection, in order to check the differentiation regulatory effect of the toll-like receptor agonist, the case without the toll-like receptor agonist-based treatment is shown as Comparative Example (pDC).

As shown in FIG. 2, we were able to confirm that the differentiation into the plasmacytoid dendritic cells was induced by applying the toll-like receptor agonist to the immature dendritic cells. We could confirm that there is no difference between differentiation efficiency in the non-treatment using the toll-like receptor agonist and differentiation efficiency in the treatment using the toll-like receptor agonist.

### [Example 2] Stimulus to isolated plasmacytoid dendritic cells and thus confirmation of secretion pattern of cytokine therefrom

In order to check the cytokine secretion pattern from the plasmacytoid dendritic cells as isolated from Example 1, the isolated cells (5 × 10⁵ cells/ml) were inoculated into a 48 well plate and treated and stimulated with ODN1826 (1 µg/ml). At 24 hours after the stimulation, supernatant was separated, and the cytokine secretion pattern was checked by ELISA (enzyme linked immunosorbent assay) and the results are shown in FIG. 4. In this connection, in order to check the effect from the treatment using the toll-like receptor agonist, in Example 1, the case in which the toll-like receptor agonist was not applied thereto during the differentiation of the immature dendritic cells is shown as the Comparative Example (pDC).

As shown in FIG. 4, in general, a type I interferon (IFN-α and IFN-β) and the typical inflammatory cytokines TNF-α and IL-12p70 were strongly induced from the plasmacytoid dendritic cells (pDC). However, when the plasmacytoid dendritic cells (TLRs-pDC) differentiated in the induced manner via the treatment with the toll-like receptor agonist (0.5 µg/ml) were stimulated with ODN1826, the expression level of the type I interferon (IFN-α and IFN-β) and the typical inflammatory cytokines TNF-α and IL-12p70 was greatly reduced. To the contrary, when the plasmacytoid dendritic cells (TLRs-pDC) differentiated in the induced manner via the treatment with the toll-like receptor agonist (0.5 µg/ml) were stimulated with ODN1826, the expression level of IL-10 as an immunosuppressive cytokine was significantly increased.

In this way, it has been shown that the plasmacytoid dendritic cells differentiated in the induced manner from the immature dendritic cells via applying the toll-like receptor agonist to the immature dendritic cells according to the present disclosure have immune tolerance unlike the conventional plasmacytoid dendritic cells.

### [Example 3] Confirmation of IL-10 secretion pattern from plasmacytoid dendritic cells induced via treatment using various toll-like receptor agonists

In order to check the immune tolerance inducing effect of various toll-like receptor agonists, differentiation into the plasmacytoid dendritic cells was induced by the same method as in Example 1. Ligands listed in Table 2 were used as the toll-like receptor agonist. From the cells obtained after 8 days of culture, the plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. ODN1826 (1 µg/ml) was applied thereto and the cells were cultured for 24 hours. Then, supernatant was separated. The secretion pattern of the immunosuppressive cytokine IL-10 was then checked by ELISA (enzyme linked immunosorbent assay). The results are shown in FIG. 5. In this connection, in order to check the effect from the treatment using the toll-like receptor agonist, the case in which the toll-like receptor agonist was not applied thereto during the differentiation of the immature dendritic cells is shown as Comparative Example (non).

**[Table 2]**

| Non | TLR2 agonist | TLR3 agonist | TLR4 agonist | TLR7 agonist | TLR9 agonist |
|---|---|---|---|---|---|
| Non-treatment | Pam3 | Poly I :C | LPS | Imiquimod | CpG-ODN |

As shown in FIG. 5, the expression level of IL-10 from only the plasmacytoid dendritic cells differentiated in the induced manner via treatments with TLR2, TLR4, TLR7 and TLR8 agonists which affects MYD88 signal among various toll-like receptor agonists was significantly increased when the plasmacytoid dendritic cells were stimulated with ODN1826.

In this case, only the treatment using the toll-like receptors which affects the MYD88 signal during differentiation of the immature dendritic cells may induce the tolerogenic plasmacytoid dendritic cells.

### [Example 4] Confirmation of cytokine secretion pattern from plasmacytoid dendritic cells according to treatment time using toll-like receptor agonist

Differentiation into the plasmacytoid dendritic cells was induced by the same method as in Example 1 in order to check the immune tolerance inducing effect of the toll-like receptor agonist according to the treatment time point. Further, the toll-like receptor agonists were applied at the start time of the differentiation (0 day treatment) and were applied on 3 days after the start of differentiation (3 day treatment). After the total 8 days culturing, the plasmacytoid dendritic cells were isolated therefrom at a purity equal to or greater than 85% using a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. ODN1826 (1 µg/ml) was applied thereto and the cells were cultured for 24 hours. Then, supernatant was separated. The secretion pattern of the cytokine was then checked by ELISA (enzyme linked immunosorbent assay). The results are shown in FIG. 6. In this connection, in order to check the effect from the treatment using the toll-like receptor agonist, the case in which the toll-like receptor agonist was not applied thereto during the differentiation of the immature dendritic cells is shown as Comparative Example (no-treatment, non).

As shown in FIG. 6, when the toll-like receptor agonist was applied at the start of differentiation (0 day treatment) or 3 days (3 days treatment) thereafter, the expression level of the type I interferon (IFN-α and IFN-β) and TNF-α and IL-12p70 as the most prominent inflammatory cytokines were inhibited as compared with the no-treatment, but the expression level of IL-10 as an anti-inflammatory cytokine was significantly increased as compared with the no-treatment.

Thus, even when the toll-like receptor agonist was applied simultaneously together with the differentiation-inducing factor FLT3L, that is, when the toll-like receptor agonist was applied at the start time of differentiation of the immature dendritic cells, the differentiation into the tolerogenic plasmacytoid dendritic cells was induced as in the case of the treatment using the toll-like receptor agonist during differentiation of the immature dendritic cells.

### [Example 5] Confirmation of expression of co-stimulation factor and MHC molecule from plasmacytoid dendritic cells induced via treatment with toll-like receptor agonist

Dendritic cells including the plasmacytoid dendritic cells may present an antigen via MHC molecules to activate T cells when detecting the external antigen, and may express co-stimulation factors such as CD80 and CD86 to stimulate interactions.

Thus, the plasmacytoid dendritic cells isolated from Example 1 were stimulated with ODN1826, and then cultured for 24 hours. Then, the expression levels of the cell surface molecules therefrom were checked. To investigate the effect of the plasmacytoid dendritic cells on the surface factor expression, anti-CD11c (PE-Cy7, BD Biosciences), and anti-PDCA-1 (PerCP-eFluor 710, ebioscience) antibodies as markers specific to the plasmacytoid dendritic cells, and anti-CD80 (v450, BD Biosciences), anti-CD86 (APC, ebioscience), anti-MHC-I (PE, ebioscience), and anti-MHC-II (APC-eFluor 780, ebioscience) antibodies as markers specific to the cell surface molecules were applied thereto at 4°C for 30 minutes. Then, the treated plasmacytoid dendritic cells were analyzed using a flow cytometer LSRFortessa x-20 (BD Biosciences)._The results are shown in FIG. 7. In this connection, in order to check the effect from the treatment using the toll-like receptor agonist, the case in which the toll-like receptor agonist was not applied thereto during the differentiation of the immature dendritic cells is shown as the Comparative Example (pDC).

As shown in FIG. 7, co-stimulation factor CD86 and MHC class II molecule presenting the exogenous antigen were expressed at high levels from the conventional plasmacytoid dendritic cells (pDC). However, co-stimulation factor CD86 and MHC class II molecule presenting the exogenous antigen were not expressed or were expressed at a lower level from the plasmacytoid dendritic cells (TLRs-pDC) differentiated in the induced manner via treatment with toll-like receptor agonist. To the contrary, one of other co-stimulatory factors, CD80 and MHC class I molecules presenting the endogenous antigen or cross-antigen were expressed at high levels from the plasmacytoid dendritic cells (TLRs-pDC) differentiated in the induced manner via treatment with toll-like receptor agonist, compared to the conventional plasmacytoid dendritic cells (pDC) without treatment with toll-like receptor agonist.

### [Example 6] Confirmation of expression of immune tolerance inducing molecule from plasmacytoid dendritic cells induced via treatment with toll-like receptor agonist

The conventional dendritic cells activate the acquired immune response by inducing T cell response via antigen presenting. However, some tolerogenic dendritic cells have been reported to suppress T cell responses. The inhibition of the immune responses has recently been reported to be achieved by the induction of various immune tolerance inducing molecules, for example, PD-L1 and IDO. Further, CCR9 + pDCs have been reported to induce various immune tolerance phenomena via strong induction of regulatory T cells.

Therefore, in order to check the expression of immune tolerance inducing molecules from the plasmacytoid dendritic cells isolated from Example 1, anti-CDllc (PE-Cy7, BD Biosciences), and anti-PDCA-1 (PerCP-eFluor 710, ebioscience) antibodies as markers specific to the plasmacytoid dendritic cells, and cell surface factors, such as, anti-PD-Ll (PE, ebioscience) and anti-CCR9 (FITC, ebioscience) as immune tolerance inducing cell surface molecules were applied thereto at 4°C for 30 minutes. Further, to determine the expression of IDO induced in the cell, fixation/permeabilization substance (BD Bioscience) were applied thereto at 4°C for 30 minutes, and anti-IDO (eFluor 660, ebioscience) was stained. The expression levels thereof were analyzed using a flow cytometer LSRFortessa x-20 and the results are shown in FIG. 8. In this connection, in order to check the effect from the treatment using the toll-like receptor agonist, the case in which the toll-like receptor agonist was not applied thereto during the differentiation of the immature dendritic cells in Example 1 is shown as the Comparative Example (pDC).

As shown in FIG. 8, when the plasmacytoid dendritic cells induced via the treatment with the toll-like receptor agonist were stimulated with ODN1826, the expression levels of CCR9 and IDO molecules therefrom were significantly increased compared with the conventional plasmacytoid dendritic cells (pDC) without treatment. When the plasmacytoid dendritic cells induced via the treatment with the toll-like receptor agonist were not stimulated with ODN1826, the expression level of CCR9 molecules therefrom was higher compared with the conventional plasmacytoid dendritic cells (pDC) without treatment.

Thus, in the treatment using the toll-like receptor agonists during differentiation of the immature dendritic cells, the differentiation into the plasmacytoid dendritic cells having immune tolerance may be induced.

### [Example 7] Confirmation of regulatory T cell inducing ability by plasmacytoid dendritic cells induced via treatment using toll-like receptor agonist

Tolerogenic plasmacytoid dendritic cells (Tolerogenic pDCs) have been reported to induce differentiation into regulatory T cells (Treg) and inhibit the activity or proliferation of effector T cells.

Therefore, we examined whether the regulatory T cells (Foxp3 + CD4 + T cells) are proliferated by the plasmacytoid dendritic cells (TLRs-pDC) differentiated in the induced manner via treatment using the toll-like receptor agonist. Specifically, T cells isolated from allogeneic mice and then stained with CellTrace (Invitrogen) were mixed and cultured with the plasmacytoid dendritic cells (TLRs-pDC) isolated from Example 1 at a mixing ratio of 5 : 1 for 5 culturing days. In this connection, the plasmacytoid dendritic cells stimulated with ODN1826 (ODN +) and the plasmacytoid dendritic cells not stimulated with ODN1826 (ODN -) were used as the plasmacytoid dendritic cells. After 5 days of culture, anti-CD4 (Percp-cy5.5, ebioscience) was applied thereto at 4°C for 30 minutes and then Foxp3/transcription factor staining buffer (eBioscience) was applied thereto at 37°C for 30 minutes. After the treatment with anti-Foxp3 (PE, ebioscience), the results were checked by the flow cytometer LSRFortessa x-20 for proliferation into the regulatory T cell. FIG. 9A shows the results. The proliferation rate of regulatory T cells was calculated and the results are shown in FIG. 9B. In this connection, in order to check the effect from the treatment using the toll-like receptor agonist, the plasmacytoid dendritic cells stimulated with ODN1826 are indicated as ODN + while conventional plasmacytoid dendritic cells not stimulated with ODN1826 are indicated as Comparative Example (pDC).

As shown in FIG. 9, the conventional plasmacytoid dendritic cells (pDC) did not induce the regulatory T cell proliferation. However, when the conventional plasmacytoid dendritic cells were subjected to the stimulation with ODN1826 (ODN +), this resulted in the inhibition of regulatory T cell proliferation. To the contrary, when the plasmacytoid dendritic cells (TLRs-pDC) differentiated in the induced manner via the toll-like receptor agonist based treatment were subjected to the stimulation with ODN1826 (ODN +), this induced the proliferation of regulatory T cell more significantly than when the plasmacytoid dendritic cells (TLRs-pDC) differentiated in the induced manner via the toll-like receptor agonist based treatment were not subjected to the stimulation with ODN1826 (ODN -).

Thus, when the plasmacytoid dendritic cells (TLRs-pDC) differentiated in the induced manner according to the present disclosure were subjected to further toll-like receptor agonist based treatment, the tolerogenic activity was induced and differentiation into the regulatory T cells was further induced.

### [Example 8] Confirmation of activity of Rv1411c protein as toll-like receptor agonist

Previous reports have shown that proteins derived from M. tuberculosis can bind to various toll-like receptors. Thus, the binding of Rv1411c protein to dendritic cells differentiated from the bone marrow of TLR2 knockout mice and wild-type mice was checked to check the activity of Rv1411c protein as a toll-like receptor agonist.

Specifically, mouse bone marrow was separated from the mouse and red blood cells were removed therefrom. For differentiation into the dendritic cells, the bone marrow was cultured for 8 days in RPMI1640 medium containing 10% FBS, 1% antibiotic and 100 ng/ml GM-CSF (granulocyte-macrophage-colony stimulating factor). After 8 days of culture, 5 µg/ml of Rv1411c protein was applied to each of the wild type dendritic cells (WT) and dendritic cells isolated from TLR2 knockout mice (TLR2 -/-) and then intermittently mixed with each other for 2 hours, to facilitate the binding of the protein to the dendritic cells. Then, after staining the mixture using antibodies having antigen-antibody specificity to His molecules labeled to the Rv1411c protein, the degree of binding therebetween was measured using a flow cytometer. The results are shown in FIG. 10.

As shown in FIG. 10, the wild-type dendritic cells (WT) were found to have increased binding to the Rv1411c protein compared to TLR2 knockout-derived dendritic cells (TLR2 -/-). When the TLR2 knockout derived dendritic cells (TLR2 -/-) was treated using the Rv1411c protein, the binding strength therebetween was confirmed to be the same as that in the untreated experimental group.

Thus, it was confirmed that the Rv1411c protein binds to the TLR2 receptor and has activity as the TL2 agonist.

### [Example 9] Confirmation of inducing ability of differentiation into tolerogenic plasmacytoid dendritic cells by Rv1411c protein

Differentiation into the tolerogenic plasmacytoid dendritic cells was induced by the same procedure as in Example 1. The toll-like receptor agonist employed the Rv1411c protein (0.1 µg/ml, 0.5 µg/ml), whose activity as the toll-like receptor agonist was confirmed in Example 8. After 8 days of culture, plasmacytoid dendritic cells were separated at a purity of 85% or greater using a plasmacytoid dendritic cell separation kit (Miltenyi Biotec, Auburn, CA) and a magnetic cell sorting system (Vario MACS: Miltenyi Biotec, Auburn, CA). The separated cells (5 × 10⁵ cells/ml) were inoculated into a 48 well plate. After treatment of the cells with ODN1826 (1 µg/ml), the cells were cultured for 24 hours. After culturing, the supernatant was separated and then the cytokine secretion pattern was checked via Enzyme Linked Immunosorbent Assay (ELISA). The result is shown as a graph in FIG. 11. In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC).

As shown in FIG. 11, when the plasmacytoid dendritic cells (Rv1411c (0.1 µg/ml)-pDC, Rv1411c (0.5 µg/ml)-pDC) treated with the Rv1411c protein were stimulated with ODN1826, the expression of the type I interferon (IFN-α and IFN-β) and TNF-α and IL-12p70, as typical inflammatory cytokines as strongly induced from the plasmacytoid dendritic cells was drastically reduced in a concentration-dependent manner, while the expression level of IL-10, as an immunosuppressive cytokine has a marked increase in a concentration-dependent manner.

These results suggest that plasmacytoid dendritic cells treated with Rv1411c protein have the immune tolerance which is not the case for conventional plasmacytoid dendritic cells. The level of the immune tolerance was increased in proportion to the concentration of the Rv1411c protein.

### [Example 10] Confirmation of expression of co-stimulation factor, MHC molecule and immune tolerance inducing molecule from plasmacytoid dendritic cells differentiated in induced manner via treatment with Rv1411c protein

In Example 9, the expression patterns of the surface molecules and enzymes from the plasmacytoid dendritic cells differentiated in induced manner via the treatment using the Rv1411c protein were checked.

First, to investigate the effect of the plasmacytoid dendritic cells on the surface factor expression, anti-CD11c (PE-Cy7, BD Biosciences), and anti-PDCA-1 (PerCP-eFluor 710, ebioscience) antibodies as markers specific to the plasmacytoid dendritic cells, and anti-CD80 (v450, BD Biosciences), anti-CD86 (APC, ebioscience), anti-MHC-I (PE, ebioscience), anti-MHC-II (APC-eFluor 780, ebioscience), anti-PD-L1(PE, ebioscience) and anti-CCR9 (FITC, ebioscience) antibodies as markers specific to the cell surface factors were applied thereto at 4°C for 30 minutes. Further, to determine the expression of IDO induced in the cell, fixation/permeabilization substance (BD Bioscience) were applied thereto at 4°C for 30 minutes, and anti-IDO (eFluor 660, ebioscience) was stained after treatment. The expression levels thereof were analyzed using a flow cytometer LSRFortessa x-20 and the results are shown in FIG. 12. In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC) as non-treated with the Rv1411c protein.

As shown in FIG. 12, the co-stimulatory factor CD86 and MHC class II molecule presenting exogenous antigen are expressed at high levels from the conventional plasmacytoid dendritic cells (pDC) as non-treated with the Rv1411c protein during the ODN1826 stimulus thereto. However, the co-stimulatory factor CD86 and MHC class II molecule presenting exogenous antigen are expressed at very lower levels or are not expressed from the plasmacytoid dendritic cells (Rv1411c-pDC) as differentiated in the induced manner via the treatment with the Rv1411c protein during the ODN1826 stimulus thereto.

To the contrary, CD80 as one of the other co-stimulatory factors, and an MHC class I molecule presenting the endogenous or cross-antigen was expressed at higher levels from the plasmacytoid dendritic cells (Rv1411c-pDC) as differentiated in the induced manner via the treatment with the Rv1411c protein than from the conventional plasmacytoid dendritic cells (pDC).

Further, the expression levels of PD-L1, CCR9, and IDO molecules from the plasmacytoid dendritic cells (Rv1411c-pDC) as differentiated in the induced manner via the treatment with the Rv1411c protein were significantly increased during the ODN1826 stimulus thereto (ODN+) compared to the ODN1826 non-stimulus (ODN-). Further, the expression level of the CCR9 and PD-L1 from the plasmacytoid dendritic cells (Rv1411c-pDC) as differentiated in the induced manner via the treatment with the Rv1411c protein during the ODN1826 non-stimulus thereto (ODN-) are higher than the expression levels thereof from the conventional plasmacytoid dendritic cells (pDC).

### [Example 11] Inhibition of T cell activation by plasmacytoid dendritic cells differentiated in the induced manner via treatment with Rv1411c protein

The most important feature of the tolerogenic plasmacytoid dendritic cells in vivo is to inhibit the activity of T lymphocytes. The following experiment was conducted to check the effect of the plasmacytoid dendritic cells differentiated in the induced manner via the treatment using the Rv1411c protein in Example 9 on the proliferation and activity of T cells.

Specifically, T cells (1.5 × 10⁵ cells/well) as isolated from allogeneic mice and stained with a CellTrace (Invitrogen) were stimulated with 1 × PMA/Ionomycin (ebioscience). This treatment of the PMA/Ionomycin increases the proliferation rate of T lymphocytes, thereby promoting the secretion of interferon gamma (IFN-gamma). When these reactions were carried out, T cells were mixed at a mixing ratio of 1 : 5 with the plasmacytoid dendritic cells (Rv1411c-pDC) differentiated in the induced manner via the treatment using the Rv1411c protein or the conventional plasmacytoid dendritic cells (pDC) and then the mixture was cultured for 3 days. After 3 days, anti-CD4 (Percp-cy5.5, ebioscience) and anti-CD8 (Percp-cy5.5, ebioscience) were applied thereto at 4°C for 30 minutes. The treated mixture was stained. The proliferation of T cells was analyzed by a flow cytometer LSRFortessa x-20 and the results are shown in FIG. 13. Further, the supernatant obtained after 3 days of culture was separated, and then the secretion pattern of IFN-gamma was checked via an Enzyme Linked Immunosorbent Assay (ELISA) and the results are shown in FIG. 14. In this connection, in order to check the effect from the treatment using the Rv1411c protein, the plasmacytoid dendritic cells treated with Rv1411c protein is indicated as Rv1411c-pDC, and the conventional plasmacytoid dendritic cells stimulated with ODN1826 (ODN +) and not stimulated with ODN1826 (ODN -) are indicated as Comparative Example (pDC) as non-treated with the Rv1411c protein.

As shown in FIG. 13, both the plasmacytoid dendritic cells (Rv1411c-pDC) differentiated in the induced manner via the treating using the Rv1411c protein and the conventional plasmacytoid dendritic cells (pDC) induced T cell proliferation. However, the plasmacytoid dendritic cells (Rv1411c-pDC) differentiated in the induced manner via the treating using the Rv1411c protein has the lower induction ability of the proliferation of CD4 + T cells compared to that of the conventional plasmacytoid dendritic cells (pDC).

In addition, as shown in FIG. 14, the plasmacytoid dendritic cells (Rv1411c-pDC) differentiated in the induced manner via the treating using the Rv1411c protein has a lower level of secretion of IFN-gamma compared to that of the non-treated conventional plasmacytoid dendritic cells (pDC).

### [Example 12] Control of regulatory T cell differentiation by plasmacytoid dendritic cells differentiated in induced manner via treating using Rv1411c protein

T cells as isolated from allogeneic mice and stained with a CellTrace (Invitrogen) were mixed at a mixing ratio of 10 : 1, 5 : 1 or 1 : 1 with the plasmacytoid dendritic cells (Rv1411c-pDC) differentiated in the induce manner via the treatment using the Rv1411c protein or the conventional plasmacytoid dendritic cells (pDC) and then the mixture was cultured for 5 days. In this connection, the plasmacytoid dendritic cells were stimulated with ODN1826 (ODN +) and unstimulated with ODN1826 (ODN -). On the fifth day after the incubation, anti-CD4 (Percp-cy5.5, ebioscience) was applied thereto at 4°C for 30 minutes. Then, Foxp3/transcription factor staining buffer (ebioscience) was applied thereto at 37°C for 30 minutes. Then, the mixture was stained with anti-Foxp3 (PE, ebioscience). The proliferation into the regulatory T cells was checked using flow cytometer LSRFortessa x-20. The results are shown in FIG. 15A. Further, the proliferation rate of regulatory T cell was calculated and the results are shown in FIG. 15B.

As shown in FIG. 15, the conventional plasmacytoid dendritic cells (pDC) did not induce regulatory T cell proliferation. When the stimulation with ODN1826 was applied to pDC, this leads to the inhibition of regulatory T cell proliferation. To the contrary, when the plasmacytoid dendritic cells (Rv1411c-pDC) differentiated in the induced manner via treatment with Rv1411c protein was subjected to the stimulation with ODN1826 (ODN +), this induced proliferation of regulatory T cell more significantly than when the plasmacytoid dendritic cells (Rv1411c-pDC) was unstimulated (ODN -). As the ratio of the plasmacytoid dendritic cells (Rv1411c-pDC) to T cells increased, we could check that the proliferation rate of regulatory T cells further increased.

Thus, when the toll-like receptor agonist was further applied to the tolerogenic plasmacytoid dendritic cell differentiated in the induced manner in accordance with the present disclosure, the tolerogenic capacity may be activated to allow the regulatory T cell proliferation to be further effectively induced.

### [Example 13] Inhibition of effector T cell activity by plasmacytoid dendritic cells differentiated in the induced manner via treatment with Rv1411c protein

Regulatory T cells have been reported to induce the tolerogenic dendritic cells and inhibit the proliferation of other T cells. Therefore, we examined the activity of the T cells differentiated in the manner induced by the Rv1411c protein-based treatment according to Example 12 as a regulatory T cell.

First, the spleen of the mouse was separated and the red blood cells were removed therefrom. CD4 +, CD25-effector T cells were isolated using a magnetic cell sorting system (MACS) and stained with a violet proliferation dye to determine the degree of proliferation. Then, the differentiated T cells were incubated together with CD4 +, CD25-effector T cells for 2 days in wells coated with anti-CD3e and anti-CD28 antibodies. As a result, the change in T cell proliferation ability was measured based on the ratio of primed T cells to CD25-effector T cells. The results are shown in FIG. 16.

As shown in FIG. 16, the only T cells induced by the ODN1826-stimulated cells (Rv1411c-pDC (ODN)) among the plasmacytoid dendritic cells differentiated in the induced manner by the Rv1411c protein-based treatment reduced the level of differentiation of effector T cells.

Thus, when the toll-like receptor agonist was further applied to the tolerogenic plasmacytoid cell differentiated in the induced manner in accordance with the present disclosure, the tolerogenic ability may be activated to allow the effector T cell activation to be effectively inhibited.

### [Example 14] Acquisition yield of plasmacytoid dendritic cells differentiated in induced manner by treatment using toll-like receptor agonist

Thigh bone marrow was collected from C57BL/6 mice using a bone marrow collecting syringe. The collected bone marrow was washed with phosphate buffered saline (PBS), and red blood cells were removed therefrom using ammonium chloride. Separated cells (5 × 10⁵ cells/well) were inoculated in a 6-well plate. Then, 1 ml of RPMI 1640 containing 10% FBS (fetal bovine serum), 2 mM L-glutamine, 100 U/ml penicillin/streptomycin, 50 µM mercaptoethanol, 0.1 mM non-essential amino acid, 1 mM sodium pyruvate and 250 ng/ml FLT3L was added thereto to initiate culture and differentiation of the cells. On the third day after the start of differentiation, Pam3 and Rv1411c protein as the toll-like receptor agonist were applied thereto at a concentration of 100 to 500 ng/ml. On 5 days from the start of differentiation, 1 ml of the RPMI 1640 was further supplemented thereto. The cells were cultured for 8 days. From the cells obtained after the incubation, plasmacytoid dendritic cells were separated at a purity equal to or greater than 85% by a plasmacytoid dendritic cell separation kit (Miltenyi Biotec, Auburn, CA) and a magnetic cell sorting system (Vario MACS: Miltenyi Biotec, Auburn, CA). The results of measuring the number (TLR agonist) of the isolated plasmacytoid dendritic cells are shown in FIG. 17. In this connection, in order to check the effect from the treatment using the toll-like receptor agonist, the non-treatment case using the toll-like receptor agonist during the differentiation of the immature dendritic cells is shown in Comparative Example (non).

As shown in FIG. 17, when the toll-like receptor agonist is applied to the immature dendritic cells during differentiation of the immature dendritic cells (TLR agonist), the number of plasmacytoid dendritic cells as differentiated in the induced manner was significantly increased compared with that of the non-treatment (Non).

### [Example 15] Allergic disease treatment effect of plasmacytoid dendritic cells differentiated in induced manner via application of toll-like receptor agonist

### 1. Preparation of ovalbumin (OVA)-derived food allergic disease animal model

In order to construct an ovalbumin (OVA)-derived food allergic disease animal model, we performed the following experiment according to the design shown in FIG. 18.

Specifically, a 4-week-old BALB/c female mouse was purchased and trained for 1 week under SPF (specific pathogen free) conditions and then tested at 5 weeks of age. 10 µg of ovalbumin (OVA) and 1 mg of aluminum hydroxide (Alum, Sigma-Aldrich Korea LTD) were injected by 100 µl into the abdominal cavity of each mouse on the 0th day and on the 7th day to induce food allergy sensitization. From 14 days to 21 days from the sensitization, boosting was performed by oral administration of ovalbumin (OVA) 10 mg per mouse for 4 times in total.

### 2. Preparation of tolerogenic plasmacytoid dendritic cells

The differentiated plasmacytoid dendritic cells induced by applying the Rv1411c protein and the conventional plasmacytoid dendritic cells were prepared in Example 9, and then were stimulated using 1 µg/ml of OVA peptide (OVA323-339 and OVA257-264) and 1 µg/ml of ODN1826 (TLR9 agonist) for 3 hours and were harvested.

To check the inhibitory effect of the food allergy via injection of the differentiated plasmacytoid dendritic cells induced by applying the Rv1411c protein, the plasmacytoid dendritic cells as obtained above were injected intravenously (i.v.) at a dose of 1 × 10⁶ cells/mouse through the vein of the prepared animal mouse model on 14-th day from the sensitization. Only phosphate buffer saline (PBS) as the negative control was injected thereto on the 14th day from the sensitization. Further, on the 28th day after the initial sensitization, OVA was administered orally in an amount of 50 mg per mouse to induce the allergic reaction.

### 3. Assessment of hypothermia and systemic anaphylaxis symptom due to allergic symptom of ovalbumin (OVA)-derived food allergic disease animal

The evaluation of systemic anaphylaxis symptom due to allergic reaction was visually observed. Thus, the disease score was checked based on the criteria listed in Table 3 below. The results are shown in FIG. 19. Further, whether hypothermia occurred was determined by measuring the change in body temperature of the rectum using a digital thermometer TESTO 925 (Testo AG, Germany) for 1 hour after inducing the allergic reaction. The results are shown in FIG. 20. The occurrence of diarrhea was evaluated and the results thereof are shown in FIG. 21.

**[Table 3]**

| Disease score | Anaphylaxis symptom |
|---|---|
| 0 | No symptom (jump freely) |
| 1 | Scratching or rubbing a portion around nose and head |
| 2 | Parts near eyes and mouth being swollen, activity loss, diarrhea |
| 3 | Breathless breathing, difficulty in breathing, cyanosis of mouth and tail |
| 4 | No activity after stimulus (no response), convulsions, trembling |
| 5 | Death |

As shown in FIG. 19, when ovalbumin (OVA)-derived food allergic reaction was induced, anaphylaxis reaction is induced and activity is reduced and parts around the eyes and mouth were swelled and the mouse was scratching and breathing breathlessly when the phosphate buffer saline (PBS) alone was injected to the mouse. However, when mice were injected with the tolerogenic plasmacytoid dendritic cells (TLRs-pDC 14d) differentiated in the induced manner via the application of the Rv1411c protein, the mouse showed little change in activity and showed only a minimal symptom of scratching the parts around the eyes and mouth. Thus, the anaphylaxis symptom was reduced compared with the conventional plasmacytoid dendritic cells (pDS) as injected thereto.

As shown in FIG. 20, when the ovalbumin (OVA)-derived food allergic reaction was induced, the hypothermia was caused by the anaphylaxis reaction in the case of the negative control (PBS) when only phosphate buffer saline was injected to the mouse. However, when mice were injected with the tolerogenic plasmacytoid dendritic cells (TLRs-pDC) differentiated in the induced manner by the application of the Rv1411c protein, the decrease in the rectal temperature was not large, and the mouse was recovered from the hypothermia faster than in the negative control (PBS).

Further, as shown in FIG. 21, regarding diarrhea occurrence, diarrhea symptom was present in all mice when administering only phosphate buffer saline (PBS) thereto. When the conventional plasmacytoid dendritic cells (pDC) were injected thereto, 80% of the mice showed diarrhea symptom. When mice were injected with the tolerogenic plasmacytoid dendritic cells (TLRs-pDC 14d) as differentiated in the induced manner by application of Rv1411c protein, the occurrence of diarrhea was significantly reduced by 40%.

Thus, the tolerogenic plasmacytoid cell differentiated in the induced manner in accordance with the present disclosure was considered to have the effect of inhibiting the induction of the ovalbumin (OVA)-derived food allergic disease.

### [Example 16] Effect of tolerogenic plasmacytoid dendritic cells on in-blood immunoglobulin in ovalbumin (OVA)-derived food allergic disease animal model

Since IgE and IgG1 antibodies are known to be the primary antibodies involved in Th2-mediated allergic responses, we examined the effect of the tolerogenic plasmacytoid dendritic cells on the changes in in-blood IgE and IgG1 concentrations in the ovalbumin (OVA)-derived food allergic disease animal models.

Specifically, after producing an ovalbumin (OVA)-derived food allergic disease animal model in the same method as in Example 15, the plasmacytoid dendritic cells as differentiated in the induced manner by application of the Rv1411c protein in Example 9 and the phosphate buffered saline (PBS) were injected to the ovalbumin (OVA)-derived food allergic disease animal model, and then 50 mg of OVA was injected thereto. After 1 hour, the blood was extracted from the mouse and the serum was separated therefrom. The in-blood concentration of the OVA-specific IgE and IgG1 in the serum was checked and the results are shown in FIG. 22.

As shown in FIG. 22, when the ovalbumin (OVA)-derived food allergic reactions were induced, in the PBS only case, the OVA-specific IgE and IgG1 concentrations increased compared to the normal group. When the tolerogenic plasmacytoid dendritic cells differentiated in the induced manner by Rvc1411c protein application was injected to the mouse, we checked that the concentration of the OVA-specific IgE and IgG1 was significantly decreased.

Thus, the tolerogenic plasmacytoid cell differentiated in the induced manner in accordance with the present disclosure may decrease the OVA-specific immunoglobulin expression occurring due to the exposure to the ovalbumin (OVA) antigen and thus may suppress the food allergic disease.

### [Example 17] Effect of tolerogenic plasmacytoid dendritic cells on Th2 cytokine production in ovalbumin (OVA)-derived food allergic disease animal model

In general, overexpression of Th2 cytokines is known to play an important role in the pathogenesis of anaphylaxis symptom. The ovalbumin (OVA)-derived food allergic disease animal model was injected with tolerogenic plasmacytoid dendritic cells. Then, we examined the changes in expression levels of interleukin-4 and interleukin-5 in serum and checked the allergic inhibitory effect based on the changes in expression levels.

Specifically, after producing an ovalbumin (OVA)-derived food allergic disease animal model in the same method as in Example 15, the plasmacytoid dendritic cells as differentiated in the induced manner by application of the Rv1411c protein in Example 9 and the phosphate buffered saline (PBS) were injected to the ovalbumin (OVA)-derived food allergic disease animal model, and then 50 mg of OVA was injected thereto. After 1 hour, the blood was extracted from the mouse and the serum was separated therefrom. The levels of interleukin-4 (IL-4) and interleukin-5 (IL-5) as the Th2 cytokines in the serum were checked and the results are shown in FIG. 23.

As shown in FIG. 23, when the ovalbumin (OVA)-derived food allergic reaction was induced, in the PBS only application case, the expression level of the Th2 cytokines, that is, the interleukin-4 and interleukin-5 increased compared with the normal group. However, when mice were injected with the tolerogenic plasmacytoid dendritic cells (TLRs-pDC 14d) differentiated in the induced manner by application of Rv1411c protein, the expression level thereof was significantly decreased.

Thus, the tolerogenic plasmacytoid cell differentiated in the induced manner by the present disclosure may inhibit the expression of Th2 cytokines when the ovalbumin (OVA)-derived food allergic reaction is induced, thereby to suppress the food allergic disease.

From the foregoing, it will be appreciated to those of ordinary skill in the art that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

## Claims

1. A method for producing a pharmaceutical composition for prevention or treatment of a hypersensitivity immune disease, the method comprising producing a tolerogenic plasmacytoid dendritic cell (pDC) by treating an immature dendritic cell with a toll-like receptor agonist.

2. The method of claim 1, wherein the toll-like receptor agonist is applied to the immature dendritic cell before differentiation initiation of or during differentiation of the immature dendritic cell, thereby to induce differentiation of the immature dendritic cell into the tolerogenic plasmacytoid dendritic cell.

3. The method of claim 1, wherein the toll-like receptor agonist is applied to the immature dendritic cell in a duration from a start time of differentiation of the immature dendritic cell to a completion time of the differentiation of the immature dendritic cell into the tolerogenic plasmacytoid dendritic cell.

4. The method of claim 2 or 3, wherein the differentiation of the immature dendritic cell into the tolerogenic plasmacytoid dendritic cell is performed using a differentiation-inducing factor.

5. The method of claim 4, wherein the differentiation-inducing factor is FMS-like tyrosine kinase 3 ligand (Flt3L).

6. The method of claim 4, wherein the differentiation-inducing factor is applied to the immature dendritic cell at one or more times.

7. The method of claim 1, wherein the toll-like receptor agonist and the differentiation-inducing factor are concurrently applied to the immune dendritic cell.

8. The method of claim 1, wherein the toll-like receptor agonist is applied to the immature dendritic cell at one or more times.

9. The method of claim 1, wherein the toll-like receptor agonist affects MyD88 (Myeloid differentiation primary response gene 88) signal.

10. The method of claim 1, wherein the toll-like receptor agonist includes at least one selected from the group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist.

11. The method of claim 10, wherein the toll-like receptor agonist further includes at least one of TLR1 and TLR6 agonists.

12. The method of claim 2, wherein the method further includes additionally applying a toll-like receptor agonist to the differentiated tolerogenic plasmacytoid dendritic cell, thereby to activate immune tolerance.

13. The method of claim 12, wherein the toll-like receptor agonist used to activate the immune tolerance is a TLR9 agonist.

14. The method of claim 1, wherein the hypersensitivity immune disease includes at least one selected from the group consisting of allergic urticaria, allergic rhinitis, allergic conjunctivitis, allergic asthma, allergic dermatitis, autoimmune hepatitis, allergic bronchopulmonary aspergillosis, and allergic stomatitis.

15. A pharmaceutical composition for prevention or treatment of a hypersensitivity immune disease, the composition comprising:
a tolerogenic plasmacytoid dendritic cell (pDC) induced by treating an immature dendritic cell with a toll-like receptor agonist.

16. The pharmaceutical composition of claim 15, wherein the tolerogenic plasmacytoid dendritic cell is differentiated from the immature dendritic cell in a manner induced by treatment of the immature dendritic cell using the toll-like receptor agonist prior to differentiation initiation of or during differentiation of the immature dendritic cell.

17. The pharmaceutical composition of claim 15, wherein the toll-like receptor agonist includes at least one selected from the group consisting of a TLR2 agonist, a TLR4 agonist, a TLR5 agonist, a TLR7 agonist, a TLR8 agonist, a TLR9 agonist, a TLR11 agonist, a TLR12 agonist and a TLR13 agonist.

18. The pharmaceutical composition of claim 17, wherein the toll-like receptor agonist further includes at least one of TLR1 and TLR6 agonists.

19. The pharmaceutical composition of claim 15, wherein the hypersensitivity immune disease includes at least one selected from the group consisting of allergic urticaria, allergic rhinitis, allergic conjunctivitis, allergic asthma, allergic dermatitis, autoimmune hepatitis, allergic bronchopulmonary aspergillosis, and allergic stomatitis.

20. A cosmetic composition for prevention or amelioration of a hypersensitivity immune disease, the composition comprising:
a tolerogenic plasmacytoid dendritic cell (pDC) induced by treating an immature dendritic cell with a toll-like receptor agonist.

21. A food composition for prevention or amelioration of a hypersensitivity immune disease, the composition comprising:
a tolerogenic plasmacytoid dendritic cell (pDC) induced by treating an immature dendritic cell with a toll-like receptor agonist.
